# EUROPÄISCHE PATENTANMELDUNG

(11) **EP 4 759 142 A1**
(43) Veröffentlichungstag der Anmeldung: **17.06.2026**
(21) Anmeldenummer: 25221914.2
(22) Anmeldetag: 09.12.2025
(51) Int. Cl.: A23C 11/10, A23C 20/02, A23L 11/50, C12N 1/205, A23L 11/30

(54) **STARTERKULTUR FÜR DIE FERMENTATION EINER PFLANZLICHEN MILCHALTERNATIVE**

(30) Priorität: 13.12.2024 DE 102024137707
(71) Anmelder: VF Nutrition GmbH, 10249 Berlin (DE)
(72) Erfinder: Birke, Anna, 10245 Berlin (DE); Braunwarth, Moritz, 10119 Berlin (DE); Hartmann, Nicolas, 10245 Berlin (DE); Kellermann, Ricarda, 49326 Melle (DE); Zhang, Yifan, 10247 Berlin (DE)
(74) Vertreter: Ostertag & Partner Patentanwälte mbB

(57) **Zusammenfassung**

Es wird eine Starterkultur für die Fermentation einer pflanzlichen Milchalternative bereitgestellt, die sich insbesondere zur Herstellung eines pflanzlichen Joghurtalternativproduktes eignet. Die Starterkultur umfasst Milchsäurebakterien, die aus Hülsenfrüchtler-Pflanzenmaterial, insbesondere aus Erbsenpflanzenmaterial, isoliert sind. Vorzugsweise umfasst die Starterkultur Milchsäurebakterien der Art *Lactobacillus rhamnosus* und/oder der Art *Lactococcus lactis* ssp. *cremoris.*

## Beschreibung

Die vorliegende Erfindung betrifft eine Starterkultur für die Fermentation einer pflanzlichen Milchalternative und insbesondere für die Herstellung eines pflanzlichen Joghurtalternativproduktes, ein Verfahren zur Herstellung eines fermentierten pflanzlichen Milchalternativproduktes sowie ein fermentiertes pflanzliches Milchalternativprodukt, das unter Verwendung der Starterkultur herstellbar ist. Weiterhin betrifft die Erfindung ein Verfahren zur Bereitstellung einer Starterkultur mit Mikroorganismen für die Fermentation einer pflanzlichen Milchalternative.

### ANWENDUNGSGEBIET UND STAND DER TECHNIK

Der Bedarf an der Entwicklung von pflanzenbasierten Lebensmitteln ist in den letzten Jahren enorm gestiegen. Neben Fleischalternativen spielen dabei Milchalternativprodukte eine große Rolle. Als Basis für Milchalternativprodukte und vor allem auch für Joghurtproduktalternativen, die momentan den drittgrößten Marktanteil an pflanzenbasierten Lebensmitteln in Europa einnehmen, werden in großem Umfang Sojabohnen eingesetzt, die jedoch unter ökologischen und ethischen Gesichtspunkten umstritten sind. Eine Alternative zu sojaproteinbasierten Produkten, insbesondere im Bereich der Joghurtalternativprodukte, stellen beispielsweise Nuss-, Kokosnuss-, Reis- und Haferprodukte dar.

Die US-Patentanmeldungsschrift US 2022/0394988 A1 beschreibt ein Herstellungsverfahren für ein fermentiertes, pflanzenbasiertes Lebensmittel, bei dem für die Fermentation Lactococcus-Arten eingesetzt werden.

Die US-Patentanmeldungsschrift US 2022/0053788 A1 beschreibt ein Verfahren zur Herstellung eines fermentierten Lebensmittels auf einer pflanzlichen Basis, beispielsweise auf einer Kombination von Soja und Cashew.

Abgesehen von sojaproteinbasierten Joghurtalternativen zeichnen sich allerdings die meisten pflanzlichen Joghurtalternativen im Vergleich zu Joghurt auf Kuhmilchbasis durch einen deutlich höheren Kohlenhydratanteil und einen sehr viel geringeren Proteinanteil im Nährwertprofil aus (Boukid, F. et al. (2021) 'Vegan alternatives to processed cheese and yogurt launched in the european market during 2020: A nutritional challenge?', Foods, 10(11), pp. 1-11).

Interessante Pflanzen für die Produktion von Milchalternativprodukten sind Vertreter der Fabaceen (Hülsenfrüchtler), beispielsweise *Pisum sativum,* die gelbe Spalterbse. Die Pflanze liefert eine nährstoffreiche Proteinquelle und lässt sich umweltschonend anbauen.

Erbsen sind auch ernährungsphysiologisch interessant, da sie einen hohen Eiweißgehalt von etwa 20 % - 35 %, einen geringen Fettgehalt von etwa 0,5 % - 4,0 % und einen hohen Stärkegehalt von etwa 30 % - 48 % aufweisen (Arteaga, V. G. et al. (2021) 'Screening of twelve pea (Pisum sativum I.) cultivars and their isolates focusing on the protein characterization, functionality, and sensory profiles', Foods, 10(4)). Zusätzlich sind sie reich an freien Aminosäuren, komplexen Kohlenhydraten und Vitaminen, wie Vitamin B1 und B2 (Urbano, G. et al. (2003) 'Nutritional evaluation of pea (Pisum sativum L.) Protein diets after mild hydrothermal treatment and with and without added phytase', Journal of Agricultural and Food Chemistry, 51(8), pp. 2415-2420).

Ein anderer interessanter Vertreter der Fabaceen im Zusammenhang mit der Herstellung von pflanzlichen Joghurtalternativen ist die Ackerbohne (*Vicia faba*).

Für die Herstellung eines pflanzenbasierten Joghurtalternativproduktes wird, vergleichbar mit der Herstellung von Joghurt auf der Basis von Milch, zunächst eine pflanzliche Milchalternative auf der Basis einer pflanzlichen Proteinquelle (Pflanzenmilch) bereitgestellt. Diese Pflanzenmilch wird durch Zusetzen einer mikrobiellen Starterkultur einer Fermentation unterzogen, sodass sich eine joghurtähnliche Textur ergibt.

Fermentation ist ein lebensmitteltechnologischer Prozess, bei dem mittels anaeroben mikrobiologischen Stoffwechselvorgängen Lebensmittel haltbar gemacht werden. Dies geschieht zum einen durch die Produktion organischer Säuren durch die mikrobielle Kultur, was zu einem Absinken des pH-Werts führt (Ansäuerung). Zum anderen können einige Stämme bioaktive Stoffwechselprodukte mit antimikrobiellen Eigenschaften produzieren, die die Haltbarkeit der Produkte verlängern.

Bisher ist es üblich, bei der Fermentation von pflanzlichen Milchalternativen auf kommerzielle Starterkulturen zurückzugreifen, die auch in der klassischen Milchsäurefermentation der Milchindustrie zum Einsatz kommen. Diese Starterkulturen können "veganisiert" werden, indem die Starterkulturen im Zuge einer Umstellung auf pflanzliche Milchproduktalternativen an Pflanzenproteine "gewöhnt" werden.

Dennoch ergeben sich mit solchen veganisierten Starterkulturen Probleme bei der Fermentation von pflanzlichen Milchalternativen, insbesondere bei pflanzlichen Milchalternativen auf der Basis von Erbsen- oder Ackerbohnenproteinen. Diese Probleme wirken sich negativ auf die Produktion selbst und das sensorische Profil des Endproduktes aus. Insbesondere sind in der Produktion lange Fermentationszeiten von etwa 16 Stunden erforderlich, wohingegen die Fermentation von Joghurt aus Kuhmilch in der Regel nur acht Stunden erfordert. Weiterhin weist das resultierende Produkt nicht vergleichbar positive Eigenschaften wie ein Joghurtprodukt auf der Basis von Kuhmilch auf, sodass die Akzeptanz beim Verbraucher oftmals nicht befriedigend ist. Vor allem ist eine unzureichende Reduktion von Fehlgeschmäckern und -aromen (zusammen als *Off-Flavours* bezeichnet) festzustellen. Diese Off-Flavours haben im Endprodukt einen negativen Einfluss auf das sensorische Profil. Beispielsweise ist ein zu hoher Gehalt von Hexanal zu beobachten. Weiterhin kommt es zu einer unzureichenden Produktion von erwünschten Aromen und Geschmackstoffen, wie vor allem Acetaldehyd, Diacetyl und Acetoin. Hieraus ergibt sich im Allgemeinen die Notwendigkeit, die erwünschten Aromen und Geschmacksstoffe in Form von Zusätzen dem Produkt nachträglich beizufügen. Darüber hinaus ist ein nicht befriedigender Abbau antinutritiver sekundären Pflanzenmetaboliten, die fermentationsinhibierend wirken, festzustellen.

Insgesamt verbleiben bei der bisher üblichen Verarbeitung von erbsen- und/oder ackerbohnenbasierten Proteinquellen zu pflanzlichen Joghurtalternativen in der Regel ungewollte Aroma- und Geschmacksstoffe im Endprodukt zurück, die das sensorische Profil negativ beeinflussen. Zum anderen werden erwünschte aroma- und geschmacksaktiven Substanzen, die das typische Joghurtprofil ausmachen, entweder gar nicht oder in zu geringen Konzentrationen während der Fermentation produziert. Weiterhin kommt es bei der bisher üblichen Fermentation von pflanzlichen Milchalternativen auf der Basis von Erbsen- oder Ackerbohnenproteinen zu einer nur unzureichende Ansäuerung des Produktes. Dies ist vermutlich auf eine zu geringe Säureproduktion der Kultur bei gleichzeitiger erhöhter Pufferkapazität von Erbsen- bzw. Ackerbohnenproteinen zurückzuführen.

Es besteht daher insbesondere bei der Verwendung von erbsen- oder ackerbohnenbasierten Inhaltsstoffen ein großes Bedürfnis, die Akzeptanz der daraus erzeugten Produkte beim Verbraucher durch ein ansprechenderes Aussehen, eine verbesserte Textur und ein verbessertes Aroma- und Geschmacksprofil zu erhöhen.

### AUFGABE UND LÖSUNG

Vor diesem Hintergrund stellt sich die Erfindung die Aufgabe, eine verbesserte Möglichkeit für die Fermentation einer pflanzlichen Milchalternative, insbesondere für die Herstellung von pflanzenbasierten Joghurtalternativen, bereitzustellen. Vor allem stellt sich die Erfindung die Aufgabe, besonders geeignete Startkulturen für die Fermentation von pflanzlichen Milchalternativen bereitzustellen.

Diese Aufgabe wird durch eine Starterkultur für die Fermentation einer pflanzlichen Milchalternative gelöst, die sich dadurch auszeichnet, dass die Starterkultur Milchsäurebakterien umfasst, die aus Hülsenfrüchtler-Pflanzenmaterial isoliert sind. Vorzugsweise können die Milchsäurebakterien aus Erbsenpflanzenmaterial, insbesondere aus Erbsensamen und/oder Erbsenschoten, isoliert sein. In anderen Ausführungsformen können die Milchsäurebakterien beispielsweise aus Ackerbohnenpflanzenmaterial isoliert sein. Diese Starterkultur mit aus solchem Pflanzenmaterial isolierten Milchsäurebakterien eignet sich in besonderer Weise für die Herstellung eines pflanzlichen Joghurtalternativproduktes.

Die Erfinder konnten mit diesem Ansatz eine Starterkultur generieren, die in besonderer Weise für die Fermentation von pflanzlichen Milchalternativen, vor allem für Milchalternativen auf der Basis von Erbsen und/oder Ackerbohnenprotein, geeignet ist.

Im Gegensatz zu dem bisher üblichen Ansatz, bei dem Starterkulturen aus der konventionellen Milchindustrie an pflanzliche Proteine adaptiert werden, geht die vorliegende Erfindung den Weg, die Milchsäurebakterien für die Starterkultur direkt aus dem pflanzlichen Material zu isolieren. Nachdem das pflanzliche Material das natürliche Habitat dieser Mikroorganismen ist, sind diese Mikroorganismen in besonderer Weise an Makro- und Mikronährstoffe aus diesem Pflanzenmaterial angepasst und können somit diese auch in besonders vorteilhafter Weise verstoffwechseln. Dies führt dazu, dass die erfindungsgemäße Starterkultur *Off-Flavour-Ge*schmacksstoffe aus dem pflanzlichen Protein reduzieren kann. Gleichzeitig sorgt die Fermentation mit der erfindungsgemäßen Starterkultur für eine cremige Textur in dem fermentierten Produkt. Gleichzeitig werden durch die Fermentation mit der erfindungsgemäßen Starterkultur für das sensorische Profil relevante Stoffe produziert, sodass auf einen Zusatz von beispielsweise natürlichen Aromen verzichtet werden kann.

In besonders bevorzugter Weise handelt es sich bei den Milchsäurebakterien um Vertreter der Art *Lactobacillus rhamnosus* und/oder der Art *Lactococcus lactis* ssp. *cremoris.*

Durch die Verwendung von verschiedenen bakteriellen Stämmen, die aus dem pflanzlichen Material isoliert wurden, kann die natürliche Stoffwechselvielfalt der mikrobiellen Gemeinschaft aus dem Pflanzenmaterial bei der Fermentation einer pflanzlichen Milchalternative mit Vorteil genutzt werden. Besonders bevorzugt ist daher die Kombination von *Lactobacillus rhamnosus* und *Lactococcus lactis* ssp. *cremoris* in der erfindungsgemäßen Starterkultur.

Vorzugsweise sind die Milchsäurebakterien der Art *Lactobacillus rhamnosus* Vertreter des Stammes P11 (Hinterlegungsstelle: DSMZ, Aktenzeichen der Hinterlegung: DSM 35210).

Die Milchsäurebakterien der Art *Lactococcus lactis* ssp. *cremoris* sind vorzugsweise Vertreter des Stammes P1 (Hinterlegungsstelle: DSMZ, Aktenzeichen der Hinterlegung: DSM 35209).

Im Rahmen der Arbeiten, die dieser Erfindung zugrunde liegen, wurden verschiedene Milchsäurebakterien aus Samen von Hülsenfrüchtlern isoliert, charakterisiert und im Hinblick auf ihre Eignung als Starterkultur, insbesondere für die Herstellung eines Joghurtalternativproduktes auf der Basis von Hülsenfrüchtlerprotein, untersucht.

Als phänotypischen Eigenschaften der Bakterienstämme wurde insbesondere das Wachstum auf und die Verwertung von Erbsen- bzw. Ackerbohnenprotein untersucht. Weiterhin wurde eine Verdickung der Matrix durch Fermentation und eine Ansäuerung der Matrix betrachtet.

Zudem wurde der Abbau von *Off-Flavour-Geschmackstoffen,* vor allem von Hexanal, und die Produktion von sogenannten "Joghurtaromen" miteinbezogen.

Bei diesen Arbeiten wurde ein *Lactobacillus rhamnosus*-Stamm (Stamm P11, Hinterlegungsstelle: DSMZ, Aktenzeichen der Hinterlegung: DSM 35210) identifiziert, der aus einer frischen Zuckererbsenschote isoliert worden war. Dieser Stamm zeichnet sich dadurch aus, dass er hervorragend auf Medium wächst, das hauptsächlich Erbsen- oder Ackerbohnenproteinisolat enthält. In einem Verdickungstest hatte dieser Stamm unter fast allen getesteten Bedingungen zu einer Verdickung der Matrix geführt, wobei dies nicht auf die Produktion von Säure zurückzuführen ist, sondern auf die Produktion von sogenannten Protein-Quervernetzern. Einer dieser Quervernetzer ist Acetaldehyd, was zum einen ein Joghurtaroma, zum anderen das Vorläufermolekül der beiden Joghurtaromastoffe Diacetyl und Acetoin ist.

Weiterhin konnten die Erfinder zeigen, dass der Stamm P11 während der Fermentation Hexanal reduziert, auch in einer Co-Kultur mit anderen ansäuernden Bakterienarten. Vor allem Hexanal und auch andere Aldehyde sind bei herkömmlichen pflanzlichen Milchalternativprodukten für *Off-Flavour*-Geschmäcker, insbesondere für einen bohnigen Geschmack, mitverantwortlich. Durch die Verringerung des Hexanal-Gehalts infolge der Fermentation mit der erfindungsgemäßen Starterkultur kann daher der resultierende Geschmack des fermentierten Produktes gegenüber herkömmlich produzierten Milchalternativprodukten wesentlich verbessert werden.

Weiterhin konnte gezeigt werden, dass der Stamm P11 in einem Agar-basierten Assay Phytinsäure degradiert. Bei Phytinsäure handelt es sich um eine bioaktive Substanz, die unter anderem in Hülsenfrüchten vorhanden ist. Aufgrund ihrer komplexbildenden Eigenschaften kann sie die Aufnahme von mit der Nahrung aufgenommenen Mineralstoffen beim Mensch hemmen, sodass Phytinsäure ernährungsphysiologisch nachteilig sein kann. Der Abbau dieser bioaktiven Pflanzensubstanz durch den Stamm P11 ist daher sehr vorteilhaft für das resultierende Milchalternativprodukt.

Im Zuge dieser Arbeiten wurden mit allen isolierten Bakterienstämmen Probefermentationen durchgeführt und sensorisch analysiert. Hierbei hatte sich neben dem Stamm P11 von *Lactobacillus rhamnosus* ein weiterer Stamm (P1) als sensorisch besonders vorteilhaft herausgestellt.

Die Bakterienart dieses Stammes P1 (Hinterlegungsstelle: DSMZ, Aktenzeichen der Hinterlegung: DSM 35209) wurde als *Lactococcus lactis* ssp. *cremoris* identifiziert.

Durch eine Kombination der Stämme P1 und P11 in einer Starterkultur können daher besonders gute Ergebnisse bei der Fermentation erreicht werden.

In einer weiteren, ganz besonders bevorzugten Ausgestaltung der erfindungsgemäßen Starterkultur umfasst die Starterkultur zusätzlich Milchsäurebakterien der Art *Streptococcus thermophilus.* In diesem Zusammenhang sind ansäuernde Stämme und vorzugsweise stark ansäuernde Stämme von *Streptococcus thermophilus* besonders bevorzugt.

Die Untersuchungen der Erfinder hatten gezeigt, dass die analysierten Stämme von *Lactobacillus rhamnosus* und *Lactococcus lactis* ssp. *cremoris* in Kombination mit einem ansäuernden Stamm von *Streptococcus thermophilus* eine Milchigkeit bei dem fermentierten Milchalternativprodukt bewirkt, die einem Fermentationsprodukt mit herkömmlichen Starterkulturen deutlich überlegen ist.

Vorzugsweise ist die pflanzliche Milchalternative auf der Basis von Erbsenprotein und/oder Ackerbohnenprotein hergestellt.

Die erfindungsgemäße Starterkultur setzt sich in ganz besonders bevorzugten Ausführungsformen aus *Lactobacillus rhamnosus* P11 (Funktion: *Off-Flavour* Reduktion und Abbau antinutritiver Inhaltsstoffe, Verdickung durch Quervernetzer-Produktion), *Lactococcus lactis ssp. cremoris* P1 (Funktion: Produktion von Milch- und Joghurtaromen) und *Streptococcus thermophilus* (Ansäuerung). Diese Starterkultur eignet sich in ganz besonderer Weise für die Fermentation von pflanzlichen Milchalternativen zur Herstellung von Joghurtalternativprodukten auf der Basis von Erbsen- und/oder Ackerbohnenproteinen.

Der ansäuernde Stamm von *Streptococcus thermophilus* kann gegebenenfalls aus Milch oder einem anderen Lebensmittel isoliert sein. Es kann sogar besonders vorteilhaft sein, dass als ansäuernde Stamm von *Streptococcus thermophilus* ein traditioneller Joghurtstamm aus einer herkömmlichen Starterkultur für Milchprodukte verwendet wird, um in dieser Co-Kultur für eine besonders gute Ansäuerung bei einer Fermentation mit der erfindungsgemäßen Starterkultur zu sorgen.

Die Erfindung umfasst weiterhin ein Verfahren zur Herstellung eines fermentierten pflanzlichen Milchalternativproduktes. Diese Verfahren zeichnet sich dadurch aus, dass das pflanzliche Milchalternativprodukt unter Verwendung einer Starterkultur gemäß der obigen Beschreibung fermentiert ist. Vorzugsweise umfasst die Starterkultur dabei Milchsäurebakterien der Art *Lactobacillus rhamnosus* und/oder der Art *Lactococcus lactis* ssp. *cremoris* und vorzugsweise zusätzlich Milchsäurebakterien der Art *Streptococcus thermophilus.* Bei den Milchsäurebakterien der Art *Lactobacillus rhamnosus* handelt es sich vorzugsweise um Vertreter des Stammes P11. Bei den Milchsäurebakterien der Art *Lactococcus lactis* ssp. *cremoris* handelt es sich vorzugsweise um Vertreter des Stammes P1, wobei sowohl der Stamm P11 als auch der Stamm P1 aus Pflanzenmaterial von Hülsenfrüchtlern isoliert ist.

In besonders bevorzugten Ausführungsformen des Herstellungsverfahrens ist das herzustellende pflanzliche Milchalternativprodukt ein Joghurtalternativprodukt.

In vorteilhaften Ausführungsformen des Herstellungsverfahrens umfasst das Verfahren die folgenden Verfahrensschritte:
a. Bereitstellen einer pflanzlichen Milchalternative,
b. Zusetzen der Starterkultur zu der pflanzlichen Milchalternative,
c. Fermentation der pflanzlichen Milchalternative,
d. Gegebenenfalls weitere Bearbeitung des pflanzlichen Milchalternativproduktes,
e. Abfüllung und Verpackung des pflanzlichen Milchalternativproduktes.

Vorzugsweise handelt es sich bei der pflanzlichen Milchalternative um eine Milchalternative auf der Basis von Erbsenprotein und/oder Ackerbohnenprotein.

Die Fermentation erfolgt vorzugsweise bei einer Temperatur in einem Bereich zwischen 35 und 45 °C. Besonders bevorzugt ist eine Temperatur von 40 °C.

Die Fermentation wird vorzugsweise für eine Zeitdauer von 5 bis 20 Stunden, vorzugsweise für eine Zeitdauer von 8 bis 16 Stunden durchgeführt.

In besonders bevorzugten Ausführungsformen erfolgt die Fermentation bzw. die Inkubation bei 40 °C für 8 bis 16 Stunden.

Beispielsweise kann für die Bereitstellung der pflanzlichen Milchalternative eine Joghurtbasis mit einem Anteil an Erbsen- und/oder Ackerbohnenprotein von 5 % hergestellt werden. Diese Joghurtbasis bzw. die pflanzliche Milchalternative wird pasteurisiert. Anschließend wird die erfindungsgemäße Starterkultur zugesetzt. Die Fermentation erfolgt beispielsweise bei 40 °C für 8 bis 16 Stunden ohne Rühren. Gegebenenfalls kann gerührt werden, um eine für Joghurtprodukte typische Konsistenz zu erreichen. Durch die Fermentation wird das Produkt angesäuert. Sollte beispielsweise aus lebensmittelrechtlichen Gründen eine weitere Ansäuerung erforderlich sein, kann gegebenenfalls noch Säure zugesetzt werden. Beispielsweise kann es sinnvoll sein, dass der pH-Wert auf pH 4,5 oder niedriger abgesenkt wird, sofern diese Ansäuerung nicht durch die Fermentation erreicht wurde. Anschließend kann das Endprodukt, gegebenenfalls nach weiterer Bearbeitung (z.B. Zusatz von Fruchtkomponenten oder ähnliches), abgefüllt werden. Das Endprodukt wird zweckmäßigerweise gekühlt, um eine weitere Aktivität der Joghurtkulturen zu minimieren.

Weiterhin umfasst die Erfindung ein fermentiertes pflanzliches Milchalternativprodukt, das sich dadurch auszeichnet, dass das fermentierte pflanzliche Milchalternativprodukt unter Verwendung einer Starterkultur gemäß der obigen Beschreibung fermentiert ist. Vorzugsweise umfasst die Starterkultur dabei Milchsäurebakterien der Art *Lactobacillus rhamnosus* und/oder der Art *Lactococcus lactis* ssp. *cremoris* und vorzugsweise zusätzlich Milchsäurebakterien der Art *Streptococcus thermophilus.* Bei den Milchsäurebakterien der Art *Lactobacillus rhamnosus* handelt es sich vorzugsweise um Vertreter des Stammes P11. Bei den Milchsäurebakterien der Art *Lactococcus lactis* ssp. *cremoris* handelt es sich vorzugsweise um Vertreter des Stammes P1.

In besonders bevorzugten Ausführungsformen des pflanzlichen Milchalternativproduktes ist das pflanzliche Milchalternativprodukt ein Joghurtalternativprodukt.

Vorzugsweise basiert das Milchalternativprodukt und insbesondere das Joghurtalternativprodukt auf einer Milchalternative auf der Basis von Erbsenprotein und/oder Ackerbohnenprotein.

Durch die Verwendung der erfindungsgemäßen Starterkultur bei der Herstellung des pflanzlichen Milchalternativproduktes weist das Milchalternativprodukte verschiedene, sehr vorteilhafte Merkmale auf. Insbesondere zeichnet sich das fermentierte pflanzliche Milchalternativprodukt durch eine Verdickung und/oder eine Ansäuerung und/oder einen reduzierten Gehalt von *Off-Flavour-Geschmacksstoffen* und/oder einen erhöhten Gehalt von Milch- und/oder Joghurt-Aromastoffen und/oder einen reduzierten Gehalt von antinutritiven Inhaltsstoffen aus.

Bei den *Off-Flavour-Geschmacksstoffen,* die durch die Fermentation mit der erfindungsgemäßen Starterkultur im Vergleich mit einer Fermentation mit herkömmlichen Starterkulturen reduziert werden können, handelt es sich insbesondere um Hexanal.

Die Milch- und/oder Joghurt-Aromastoffe, die im Vergleich mit herkömmlichen Starterkulturen im resultierenden Produkt einen erhöhten Gehalt aufweisen können, handelt es sich insbesondere um die Aromastoffe Acetaldehyd und/oder Diacetyl und/oder Acetoin.

Bei den antinutritiven Inhaltsstoffen, die im Vergleich mit herkömmlichen Starterkulturen im resultierenden Produkt einen reduzierten Gehalt aufweisen können, handelt es sich insbesondere um fermentationsinhibierende sekundäre Pflanzenmetabolite, beispielsweise um Phytinsäure.

In ganz besonders bevorzugten Ausführungsformen des fermentierten pflanzliches Milchalternativproduktes ist das Milchalternativprodukt frei von zugesetzten Aromastoffen. Durch die besonderen beschriebenen Vorteile, die durch die Verwendung der erfindungsgemäßen Starterkultur bei der Fermentation erreicht werden, ist es möglich, vorzugsweise vollständig auf den Zusatz von Aromastoffen zu verzichten, da das resultierende Produkt auch ohne solche Zusätze ein für den Verbraucher sehr angenehmes sensorisches Profil aufweist.

Vorzugsweise sind in dem fermentierten pflanzlichen Milchalternativprodukt insgesamt über 10⁸ Mikroorganismen vorhanden, wobei vorzugsweise *Lactobacillus rhamnosus* und *Streptococcus thermophilus* etwa in einem Verhältnis von 1:1 vorhanden sind.

Die Erfinder konnten in Versuchen beobachten, dass bei einer Fermentierung mit der erfindungsgemäßen Starterkultur eine stärkere Ansäuerung des Produkts als mit einer herkömmlichen Starterkultur auftrat. Dies stellt einen weiteren Vorteil der erfindungsgemäßen Starterkultur dar, da die Ansäuerung eine wesentliche Funktion der Fermentation ist.

Die Erfindung umfasst schließlich ein Verfahren zur Bereitstellung einer Starterkultur mit Mikroorganismen, insbesondere mit Milchsäurebakterien und/oder Hefen, vorzugsweise mit Milchsäurebakterien, für die Fermentation einer pflanzlichen Milchalternative auf der Basis einer pflanzlichen Proteinquelle aus einem definierten pflanzlichen Material. Diese Verfahren zeichnet sich dadurch aus, dass das definierte pflanzliche Material für die Isolierung der Mikroorganismen eingesetzt wird.

In besonders vorteilhaften Ausgestaltungen des Verfahrens sind die folgenden Verfahrensschritte umfasst:
a. Bereitstellen einer Probe des definierten pflanzlichen Materials,
b. Anreicherung und Isolierung von Mikroorganismen, insbesondere von grampositiven Bakterien, aus der Probe des pflanzlichen Materials,
c. Gegebenenfalls genotaxonomische Identifizierung der Mikroorganismen,
d. Phänotypische Charakterisierung der Mikroorganismen,
e. Durchführung von Probefermentationen der pflanzlichen Milchalternative mit den isolierten Mikroorganismen,
f. Auswahl und Zusammenstellung von geeigneten Mikroorganismen auf der Basis der Ergebnisse der Probefermationen für die Bereitstellung einer Starterkultur.

In bevorzugten Ausführungsformen des Verfahrens umfasst die phänotypische Charakterisierung der Mikroorganismen mindestens einen der folgenden Punkte:
a. Verdickung einer Matrix,
b. Ansäuerung einer Matrix,
c. Abbau von *Off-Flavour-Geschmacksstoffen,* insbesondere von Hexanal,
d. Produktion von Milch und/oder Joghurt-Aromastoffen, insbesondere von Acetaldehyd und/oder Diacetyl und/oder Acetoin,
e. Verwertbarkeit von verschiedenen Zuckerquellen,
f. Einfluss von verschiedenen Zuckerquellen auf eine Metabolitenproduktion.

In besonders vorteilhaften Ausführungsformen des Verfahrens handelt es sich bei dem definierten pflanzlichen Material um Pflanzenmaterial eines Vertreters der Fabaceen (Hülsenfrüchtler). Besonders bevorzugte Vertreter der Fabaceen sind hierbei *Pisum sativum* und/oder *Vicia faba.* Als Pflanzenmaterial für die Isolierung und/oder Charakterisierung eignen sich beispielsweise die Erbsenschoten oder die Bohnen dieser Arten.

Nachfolgend werden Ausführungsbeispiele der Erfindung anhand der Zeichnungen näher erläutert. Die einzelnen Merkmale können jeweils für sich oder in Kombination miteinander verwirklicht sein.

### KURZE BESCHREIBUNG DER ZEICHNUNGEN

In den Zeichnungen zeigen:
- Figur 1: Übersicht über die Arbeitsschritte zur Isolierung der Mikroorganismen für die Starterkultur;
- Figur 2: Flussdiagramm der Arbeitsschritte mit Beispielbildern zur Isolierung und Identifizierung von Milchsäurebakterien aus Erbsen- und Bohnenproben. Kb: Kilobasen; NTC: Negativkontrolle der PCR (*non-template control*).
- Figur 3: Agarosegel (1,0 %) mit Banden, die bei der PCR mit spezifischen Primerpaaren amplifiziert wurden;
- Figur 4: Wachstum der Stämme P11 und P1 auf erbsenproteinhaltigem Medium mit verschiedenen Zuckerquellen;
- Figur 5: Schema zentraler Stoffwechselwege, in denen Pyruvat als Vorläufermolekül dient. *als*: Acetolaktat-Synthase; *ldh:* Laktatdehydrogenase; *pdh:* Pyruvatdehydrogenase;
- Figur 6: Agarosegel (2,0 %) der RT-PCR mit gDNA als Template. 1: P11-gDNA als Template; 2: P12-gDNA als Template; 3 und 4: *L. rhamnosus*-gDNA von Stämmen, die aus kommerziellen Joghurtproben isoliert wurden; NTC: Negativkontrolle der PCR (*non-template control*);
- Figur 7: Relatives Transkriptvorkommen von *ldh* und *als* in drei unabhängigen P11-Flüssigkulturen zu ausgewählten Zeitpunkten. Die Fehlerbalken zeigen die Standardab- weichung an (*=p-Wert unter 0,05). A) PPM: Erbsenmedium; B) PPMG: Erbsenmedium, 1,0 % Glukose; C) PPMF: Erbsenmedium, 1,0 % Fruktose; D) PPMS: Erbsenmedium, 2,0 % Saccharose;
- Figur 8: Repräsentative 96-well Platten mit Ackerbohnenmedium vor (A) und nach (B) Invertieren;
- Figur 9: Hexanalkonzentrationen [g/L] in den fermentierten Proben von P1 und P11 im Vergleich zu der unfermentierten Kontrolle (Medium) nach 6 Stunden Inkubation bei 40 °C; und
- Figur 10: Relative Hexanalgehalte in fermentierten Proben. JC 18: *Streptococcus thermophilus,* isoliert aus Erbsenmaterial; Ref: Probe, die mit der kommerziellen Starterkultur fermentiert wurde; Mix 1 - Mix 6: Fermentation mit verschiedenen, aus Erbsen isolierten Stämmen in Kombination mit einem schwach ansäuernden *St. thermophilus-*Stamm; Mix 1 enthält Stamm P1 und Mix 6 enthält Stamm P11.

### BESCHREIBUNG BEVORZUGTER AUSFÜHRUNGSBEISPIELE

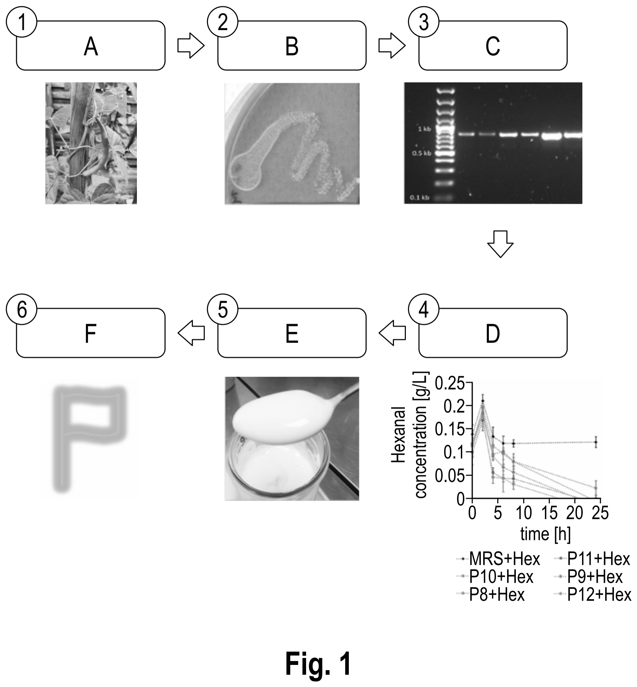

**Fig. 1** zeigt eine Übersicht der Arbeitsschritte zur Isolierung und Charakterisierung von Milchsäurebakterien aus Pflanzenmaterial mit beispielhaften Illustrationen. Mit diesem Verfahren wurden Milchsäurebakterien aus Erbsenpflanzen und Ackerbohnenpflanzen isoliert, charakterisiert und auf ihre Eignung für eine Starterkultur untersucht, die bei der Herstellung einer erbsen- oder bohnenproteinbasierenden Joghurtalternative eingesetzt werden können. Die verschiedenen untersuchten Stämme wurden mit dem Buchstaben P und fortlaufender Nummer bezeichnet.
Schritt A - Sammeln von Pflanzenmaterial
Schritt B - Anreicherung und Isolierung von Milchsäurebakterien
Schritt C - Genotaxonomische Identifizierung
Schritt D - Phänotypische Charakterisierung
Schritt E - Probefermentationen
Schritt F - Zusammenstellung der Starterkultur

### Isolierung und Identifizierung von Milchsäurebakterien aus Erbsen- und Bohnenmaterial

Für die Isolierung und Identifizierung von Milchsäurebakterien aus Erbsen- und Bohnenmaterial wurden zunächst Erbsen und Bohnen aus sieben verschiedenen Quellen gesammelt, zum Teil direkt von Erbsenfeldern aus Bayern, Nordrhein-Westfalen und Brandenburg. Aus den Quellen (Erbsen und Bohnen), wurden 13 Stämme isoliert, die fünf verschiedenen Milchsäurebakterienspezies zugehörig waren.

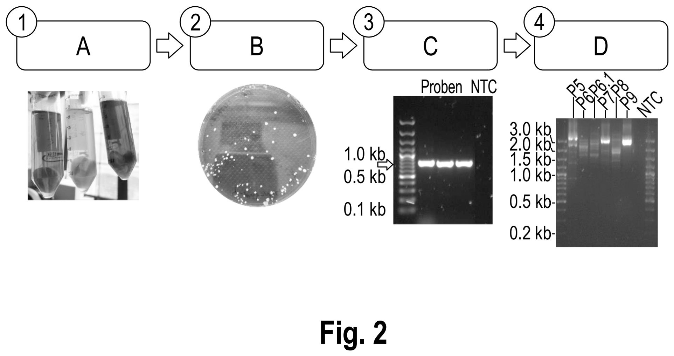

**Fig. 2** illustriert die Schritte der Isolierung und Identifizierung der Milchsäurebakterien.
Schritt A - Anreicherung
Schritt B - Selektion
Schritt C - Identifizierung der Spezies
Schritt D - Subspezies/Stamm-Unterscheidung

Anreicherung (Schritt A): Die Anreicherung der Mikroorganismen erfolgte in Flüssigmedium, in dem das Wachstum von Milchsäurebakterien begünstigt ist. Um die Mikroorganismen auf den Erbsen- und Bohnenproben anzureichern, wurden die Samen in Flüssigmedium bis zu 4 Wochen bei 15 - 18 °C unter anaeroben Bedingungen inkubiert. Um sowohl epi- als auch endophytische Bakterien anzureichern, wurden einige Flüssigkulturen mit zermahlenen Samen angesetzt. Für die Anreicherung wurden unterschiedliche Medien verwendet, insbesondere M17 Medium (links bei Schritt A der Fig. 2), Trypton-Soja Hefeextrakt Medium (TSY; Mitte bei Schritt A der Fig. 2) und De Man-Rogosa-Sharpe Medium (MRS; rechts bei Schritt A der Fig. 2).

Selektion (Schritt B): Nachdem eine sicht- und als optische Dichte messbare Trübung des Flüssigmediums erreicht war, wurden kleine Volumina auf verschiedene Agarplatten (MRS, M17, TSY, SM) ausgestrichen. Die Platten wurden vorher mit Nalidixinsäure versetzt, einem Antibiotikum, das zwar sowohl gegen gramnegative als auch grampositive Bakterien wirksam ist. In niedrigen Dosen hemmt Nalidixinsäure aber vorwiegend das Wachstum gramnegativer Bakterien. Die beispielhafte Platte im Schritt B der Fig. 2 zeigt einzelne Kolonien auf MRS-Agar. Bei einer Ausplattierung auf Medium ohne Nalidixinsäure wuchsen vorwiegend Enterobakterien und Vertreter der Gattungen Shigella und Clostridium auf den Platten.

Identifizierung der Spezies (Schritt C): Die genotaxonomische Identifizierung der Spezies wurde mittels 16S rRNA-Amplifizierung (PCR-Produkt mit 0,8 Kilobasen) durchgeführt. Die resultierenden PCR-Produkte wurden anschließend mit den gleichen Primern sequenziert (siehe A-forijiku, S., Fakorede, C. O. and Adediran, A. B. (2021) 'Strain-level Identification of Beneficial Lactobacilli of Dairy Origin using 16S rRNA Sequencing: A Biotechnology Approach', Microbiology Research Journal International, 31(2), pp. 13-21). Über einen Datenbankabgleich mit bereits sequenzierten und identifizierten Spezies wurden die isolierten Bakterien einer Spezies/Art zugeordnet. Das Agarosegel im Schritt C der Fig. 2 zeigt die PCR-Produkte (ca. 0,8 Kilobasen), die mit den 16S Primern amplifiziert wurden.

Stamm/Subspezies-Unterscheidung (Schritt D): Subspezies der gleichen Spezies wurden mit spezifischen Primern bestimmt. Die Unterscheidung mehrerer Stämme der gleichen Art erfolgte mittels *random amplification of polymeric DNA (RAPD)*-PCR. Die RAPC-PCR wurde mit dem Universalprimer M13 durchgeführt, der an verschiedenen Stellen im Genom bindet und, je nach Genomsequenz, zu distinkten Bandenmustern, sogenannten *Fingerprints,* führt (siehe Rossetti, L. and Giraffa, G. (2005) 'Rapid identification of dairy lactic acid bacteria by M13-ge-nerated, RAPD-PCR fingerprint databases', Journal of Microbiological Methods, 63(2), pp. 135-144).

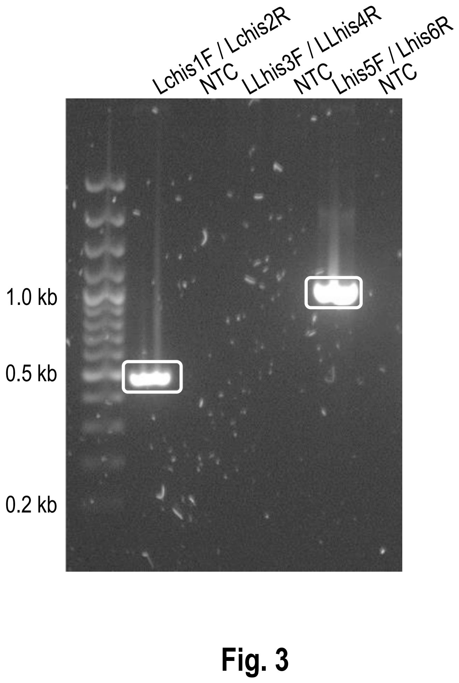

Eine der identifizierten Bakterienarten war *Lactococcus lactis,* von der drei Subspezies bekannt sind: *Lactococcus* lactis, *Lactococcus lactis* biovar. *diacetylactis* und *Lactococcus lactis* ssp. *cremoris.* Diese drei Subspezies lassen sich mit spezifischen Primerpaaren, die im Histidin-Biosynthese-Operon binden, voneinander unterscheiden (Beimfohr, C., Ludwig, W. and Schleifer, K. H. (1997) 'Rapid genotypic differentiation of Lactococcus lactis subspecies and biovar', Systematic and Applied Microbiology, 20(2), pp. 216-221). **Fig. 3** zeigt das Ergebnis dieser PCR mit spezifischen Primern. Anhand der sichtbaren Banden von ca. 0,5 kb und 1,0 kb wurde das Bakterium als *Lactococcus lactis* ssp. *cremoris* identifiziert.

### Verwertung von industriell relevanten Zuckerquellen und deren Einfluss auf die Metabolitenproduktion

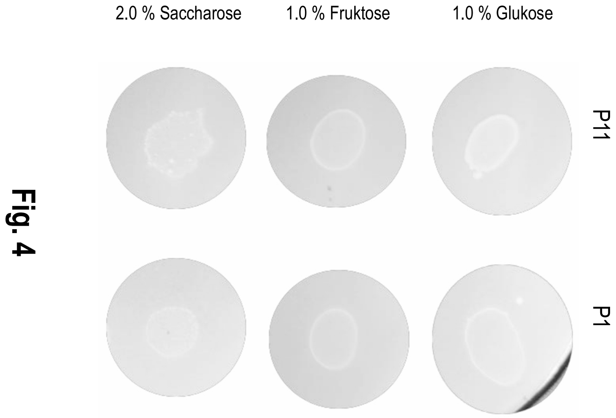

Um die Präferenz für Zuckerquellen zu untersuchen, wurde dem Nährmedium entweder Glukose (1,0 %), Fruktose (1,0 %) oder Saccharose (2,0 %) zugesetzt. Für diesen Wachstumsvergleich wurden ca. 8 x 10⁵ Zellen auf die Agarplatten getropft und zwei Wochen lang anaerob bei der industriellen Fermentationstemperatur 40 °C inkubiert. **Fig. 4** zeigt das Wachstum von P11 und P1 auf erbsenproteinhaltigem Medium mit diesen verschiedenen Zuckerquellen nach einer zweiwöchigen Inkubationsphase bei 40 °C.

Obwohl weder die getesteten Zuckerquellen noch die Fermentationstemperatur dem natürlichen ursprünglichen Habitat dieser Stämme entsprach, konnte ein gutes Wachstum bei beiden Stämmen beobachtet werden.

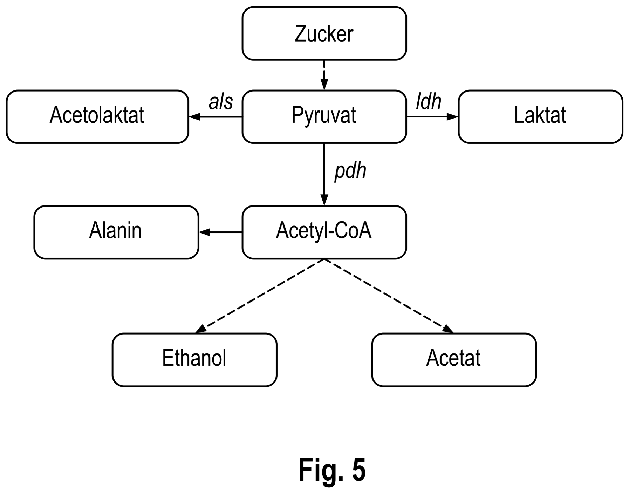

**Fig. 5** zeigt ein Schema mit zentralen Stoffwechselwegen, in denen Pyruvat als Vorläufermolekül dient (*als*: Acetolaktat-Synthase; *ldh:* Laktatdehydrogenase; *pdh:* Pyruvatdehydrogenase). Die direkten Stoffumwandlungen sind mit ununterbrochenen Pfeilen dargestellt, indirekte Stoffumwandlungen sind mit unterbrochenen Pfeilen dargestellt. Dabei sind die Milchsäureproduktion (Laktatproduktion) und die Aromabildung (Bildung von Acetolaktat) konkurrierende Stoffwechselwege.

Um in Bezug auf die untersuchten Stämme zu verstehen, welche Stoffwechselwege unter welchen Bedingungen aktiv sind, wurden semi-quantitative Genexpressionsanalysen durchgeführt.

Die Gene, die ausgewählt wurden, kodieren für die L-Laktatdehydrogenase (*ldh*), Acetolaktat-Synthase (*als*) und eine Pyruvatdehydrogenase Untereinheit (*pdh*)*.* Das Schema in Fig. 5 zeigt, wie die Enzyme im Wettbewerb miteinander stehen, da Pyruvat für alle Reaktionen das Vorläufermolekül ist. Die Umwandlung von Pyruvat zu Milchsäure durch die L-Laktatdehydrogenase ist das wichtigste Mittel der Ansäuerung bei der Joghurtherstellung. Acetolaktat hingegen ist ein intermediäres Stoffwechselprodukt, das weiter zu Diacetyl und Acetoin umgewandelt werden kann, welche zentrale Aromakomponenten eines Joghurts sind. Da *L. rhamnosus* ein heterofermentatives Bakterium ist, kann die Bildung von Acetat und Ethanol nicht gänzlich ausgeschlossen werden, wenn ausreichend Pyruvat vorhanden ist. Deshalb wurde *pdh* als Schlüsselgen in die Analyse aufgenommen.

Eine Übersicht der ausgewählten Gene, die Funktion des jeweiligen Genproduktes sowie die Größe der resultierenden PCR-Produkte sind in der nachfolgenden Tabelle 1 dargestellt.

**Tabelle 1: Übersicht über ausgewählte Gene zentraler Stoffwechselwege, mit der Funktion des jeweiligen Genproduktes und der Größe des PCR- Produktes bei einer RT-PCR.**

| **Gen** | **Funktion des Genproduktes** | **Größe des PCR-Produktes** |
|---|---|---|
| *recA* | Rekombinase A | 210 Base |
| *ldh* | Lactatdehydrogenase | 381 Basen |
| *pdhA* | Pyruvatdehydrogenase E1 Komponente Alpha Untereinheit | 478 Basen |
| *als* | 2-Acetolactat-Synthase | 604 Basen |

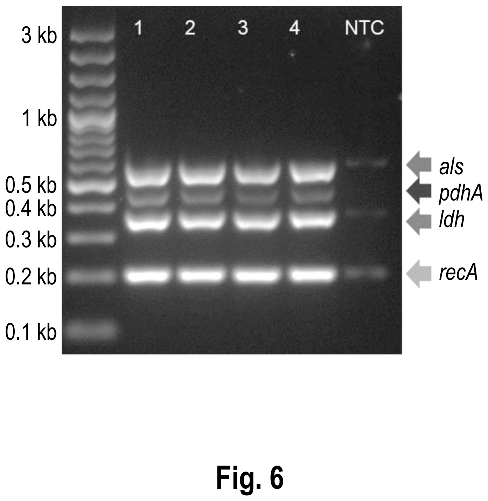

Primerpaare, die spezifisch DNA-Abschnitte innerhalb von *ldh, als* und *pdhA* amplifizieren, wurden zunächst mit genomischer DNA (gDNA) als PCR-Template getestet. **Fig. 6** zeigt die resultierenden PCR-Produkte. Hierfür wurden vier verschiedene gDNA-Proben als Template verwendet, nämlich von P11, einem weiteren Stamm P12 und von zwei *L. rhamnosus* Stämmen, die aus pflanzlichen Joghurtalternativen isoliert wurden. Wie in dem Agarosegel der Fig. 6 zu erkennen ist, sind pro Spur vier klare Banden der erwarteten Größen (siehe Tabelle 1) zu sehen. Die hellen Banden in der Spur der Negativkontrolle (NTC) sind vermutlich einem Überlaufen der Probe 4 beim Beladen des Gels zuzuschreiben.

Im nächsten Schritt wurde P11 selektiert und in den folgenden Flüssigmedien kultiviert: Erbsenproteinmedium ohne zugesetzte Kohlenhydratquelle (PPM), sowie PPM mit entweder 1,0 % Glukose (PPMG), 1,0 % Fruktose (PPMF) oder 2,0 % Saccharose (PPMS). Die Kulturen wurden jeweils in biologischen Triplikaten angesetzt. Zunächst wurden die relativen Transkriptlevel der Schlüsselgene in 24-Stunden alten Flüssigkulturen miteinander verglichen. Hierzu wurde nach 24 Stunden die RNA der Flüssigkulturen isoliert, anschließend mit einer reversen Transkriptase in cDNA umgeschrieben, um dann in einer PCR die gewünschten Produkte zu amplifizieren. Die Stärke bzw. Helligkeit der Bande auf einem Agarosegel zeigte an, wie viel der Ausgangstemplates in der Reaktion vorhanden war. Daraus lässt sich auf das relative Vorkommen des jeweiligen RNA-Transkripts schließen.

Die Helligkeit der Banden wurde in einem Bildbearbeitungsprogramm bestimmt (imageJ). Anschließend wurden die Werte der *ldh-* und *als*-Banden auf die Helligkeit der zugehörigen *recA-*Bande normalisiert. Bei *recA* handelt es sich um ein Housekeeping-Gen, dessen Expression konstant bleibt und daher üblicherweise für die Normalisierung bei semi-quantitativen Genexpressionsanalysen herangezogen wird.

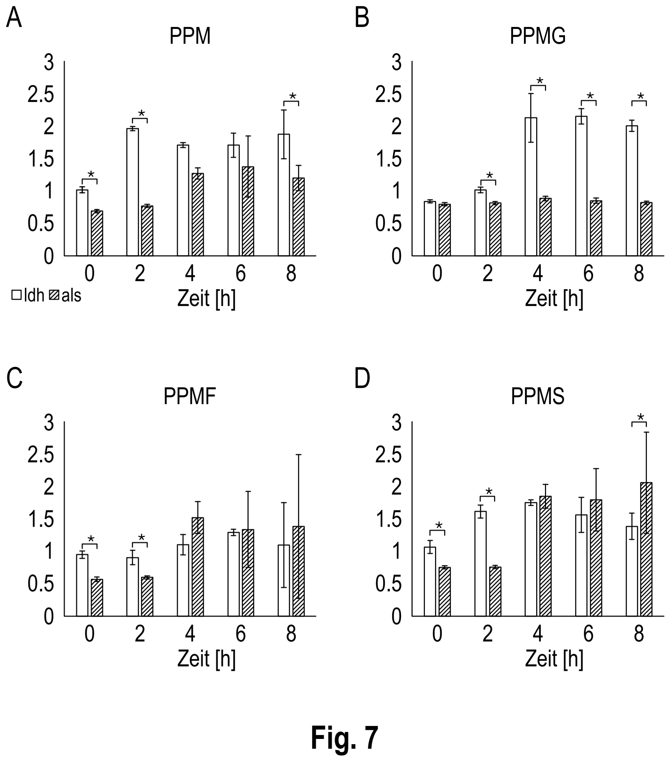

**Fig. 7** zeigt die Ergebnisse zu dem relativen Transkriptvorkommen von *ldh* und *als* in drei unabhängigen P11-Flüssigkulturen zu ausgewählten Zeitpunkten. Die RNA-Transkripte bei P11 wurden bei einer Inkubation über 24 Stunden in verschiedenen Medien untersucht (Erbsenprotein-Medium (PPM), Erbsenprotein-Medium mit 1,0 % Glukose (PPMG), Erbsenprotein-Medium mit 1,0 % Fruktose (PPMF), Erbsenprotein-Medium mit 2,0 % Saccharose (PPMS)). Statistisch signifikante Unterschiede wurden mittels Kruskal-Wallis-Test ermittelt (verwendetes Tool: https://www.statskingdom.com/kruskal-wallis-calculator.html). Eine Übersicht der ermittelten p-Werte ist in Tabelle 2 zu finden.

**Tabelle 2: Übersicht der in Kruskal-Wallis (KW)-Tests ermittelten p-Werte für die in Fig. 7 dargestellten Werte. ns: nicht signifikant.**

| **Medium** | **Zeit [h]** | **KW-Test p-Wert** |
|---|---|---|
| **PPM** | 0 | 0,00189 |
| | 2 | 0 |
| | 4 | ns |
| | 6 | ns |
| | 8 | 2,47E-06 |
| **PPMG** | 0 | ns |
| | 2 | 1,97E-07 |
| | 4 | 4,7E-06 |
| | 6 | 1,19E-11 |
| | 8 | 8,40E-12 |
| **PPMF** | 0 | 0,0000174 |
| | 2 | 0,001351 |
| | 4 | ns |
| | 6 | ns |
| | 8 | ns |
| **PPMS** | 0 | 0,00189 |
| | 2 | 0 |
| | 4 | ns |
| | 6 | ns |
| | 8 | 2,5E-06 |

Während das relative Transkriptvorkommen von *ldh* in PPM und PPMG teilweise signifikant größer war als das von *als,* war ein deutlicher Einfluss der Glukose in PPMG auf die Expression der beiden Gene erkennbar. Möglicherweise liegt eine Stimulierung der *ldh*-Expression sowie eine mögliche Unterdrückung der Expression durch eine Katabolitenrepression vor. Die Lenkung des Kohlenstoffflusses in Richtung Laktatbildung in PPMG könnte bei einer Fermentation zur Säurebildung auf Kosten der Aromabildung führen.

Die in PPMS enthaltene Saccharose wirkte sich hingegen positiv auf die *als-* und die *ldh*-Expression aus, wobei der limitierende Faktor für eine Acetolaktat- und Laktatbildung die intrazelluläre Verfügbarkeit von Pyruvat war. Das heißt, selbst wenn Saccharose die Genexpression von *ldh* und *als* anregt, bedeutet das nicht, dass insgesamt mehr Kohlenstoff umgesetzt werden kann. Saccharose ist ein Disaccharid, das zum einen in die Zelle transportiert und zum anderen hydrolysiert werden muss. Diese Schritte bestimmen maßgeblich die intrazelluläre Verfügbarkeit von Pyruvat.

Aufgrund dieser Daten wurde die Zuckerquelle in dem Joghurtalternativproduktrezept angepasst, indem eine Mischung aus Glukose und Saccharose zugegeben wird.

### Verdickung der Matrix durch Fermentation

Basierend auf der Methode, die in dem Artikel von Genet et al. (Genet, B. M. L. et al. (2023) 'Selection of proteolytic LAB starter cultures for acidification of soy based dairy alternatives', LWT, 184, p. 115082) veröffentlicht wurde, wurde die Säureproduktion der Milchsäurebakterien und die damit verbundene Verdickung der Matrix untersucht. Da die Verdickung sowohl durch die Produktion von Säure, z.B. Milchsäure, und das damit verbundene Ausfällen der im Rohmaterial enthaltenen Proteine, als auch durch die Produktion von Exopolysacchariden, eine wichtige Aufgabe der Joghurtstarterkultur ist, wurde die in dem Artikel vorgestellte Methode des "Tiltings" adaptiert und angewendet, um die Fähigkeit der Matrixverdickung der aus Erbsenmaterial isolierten Stämme zu testen. Mit der adaptierten Methode wurde in 96-Well Platten flüssiges Erbsen-(PPM) und Ackerbohnenmedium (FPM) getestet, das entweder keinen weiteren Zucker (0% Zucker) enthielt oder, analog zu den Agarplatten, Glukose (PPMG, FPMG), Fruktose (PPMF, FPMF) oder Saccharose (PPMS, FPMS) enthielt. Nach einer 14 - 16-stündigen Inkubation wurden die Platten invertiert.

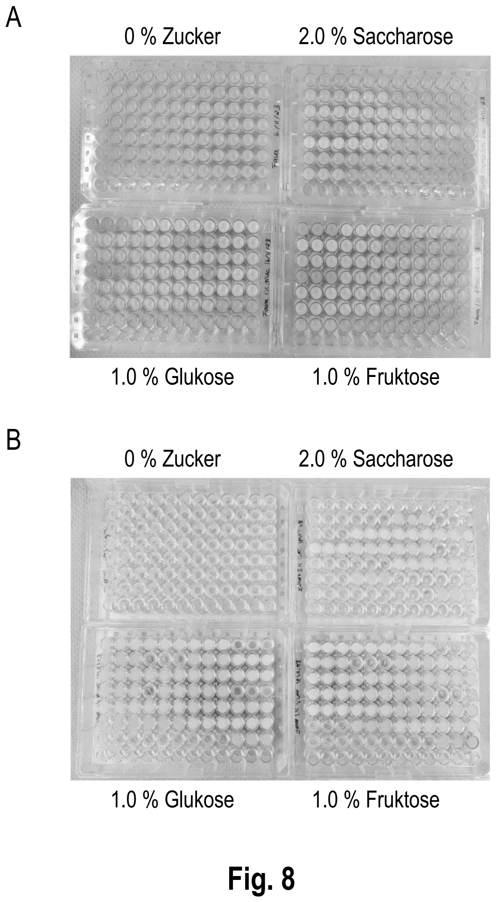

**Fig. 8** zeigt eine repräsentative 96-well Platten mit Ackerbohnenmedium (FPM) vor (A) und nach (B) dem Invertieren. Nur FPM, das im Laufe der Inkubation verdickt wurde, war nach dem Invertieren noch in den Wells zu sehen (Negativkontrolle: FPM mit Wasser). Wells, in denen das Medium nicht verdickt war, waren nach dem Invertieren leer, wie es bei allen Stämmen der Fall war, wenn kein zugesetzter Zucker im Medium vorhanden war. Mit zugesetztem Zucker waren einige Wells verdickt, was sich auch an der helleren Farbe erkennen ließ.

Der Stamm P11 verdickte die Matrix immer dann, wenn entweder Glukose oder Fruktose enthalten war. P1 hingegen verdickte die erbsenproteinhaltige Matrix nur mit zugesetzter Fruktose und die ackerbohnenproteinhaltige Matrix mit allen getesteten Zuckerquellen. Das festeste Gel produzierte P11 in PPMG. Die Verdickung kann hierbei allerdings nicht hauptsächlich auf Säurebildung zurückgeführt werden, da der pH nur minimal abgesenkt wurde. Diese Beobachtung wurde zu einem späteren Zeitpunkt auch in größerem Maßstab in den Probefermentationen bestätigt.

### Abbau von Off-Flavours (Hexanal)

Hexanal ist ein Aldehyd, das zu dem grasigen, grünen *Off-Flavour* in pflanzlichen Lebensmitteln beiträgt. Darüberhinaus kann es als Vertreter für durch Lipidoxidierung entstandene Aldehyde in Erbsen- und Ackerbohnenproteinisolaten betrachtet werden, deren *Off-Flavour* im sensorischen Gesamtprofil als störend wahrgenommen werden (siehe z. B. Roland, W. S. U. et al. (2017) 'Flavor aspects of pulse ingredients', Cereal Chemistry, 94(1), pp. 58-65).

Die Methode, die für die Messung der Verringerung des Hexanalgehalts herangezogen wurde, wurde basierend auf der von Kozaeva et al. beschriebenen Methode für die Quantifizierung von Hexanal in Flüssigmedium adaptiert (siehe Kozaeva, E. et al. (2022) 'High-throughput colorimetric assays optimized for detection of ketones and aldehydes produced by microbial cell factories', Microbial Biotechnology, 15(9), pp. 2426-2438). Hierfür wurde 0,2 g/L Hexanal dem MRS-Medium zugesetzt, beimpft und die Absorbanz bei einer Wellenlänge von 630 nm gemessen. Anhand einer Kalibriergeraden wurden die Hexanalkonzentrationen in den fermentierten Proben bestimmt. Da 630 nm keine Wellenlänge ist, bei der ausgeschlossen werden kann, dass Überreste der Kultur nach dem Abzentrifugieren oder andere Substanzen im Medium nicht auch gemessen werden, wurden zusätzlich identische Kulturen ohne Hexanal bei 630 nm analysiert.

Obwohl in den Kontrollkulturen ohne Hexanalzugabe Absorbanzwerte >0 gemessen wurden, waren die Werte über den Messzeitraum konstant (nicht abgebildet), anders als für die berechneten Hexanalwerte der fermentierten Proben. Für diese Proben war ein deutlicher Abfall nach vier Stunden gegenüber der Mediumkontrolle (MRS plus 0,15 g/L Hexanal) festzustellen. Die Differerenz der Hexanalkonzentrationen der fermentierten Proben im Vergleich mit der Mediumkontrolle wurde nach vier Stunden immer größer und war bei 24 Stunden maximal.

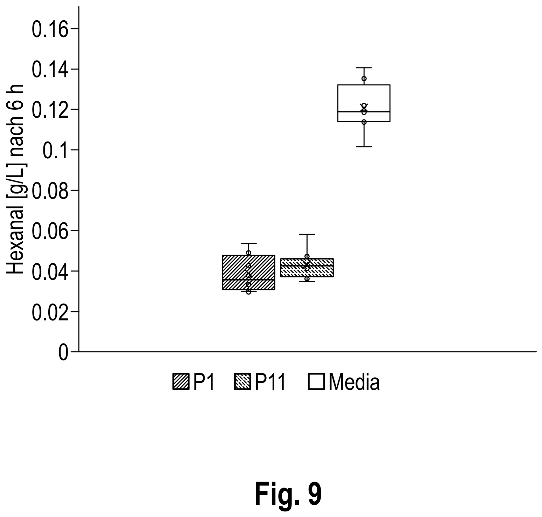

Nach 6 Stunden waren deutliche Unterschiede zwischen den Stämmen erkennbar. Wie der **Fig. 9** zu entnehmen ist, zeichnete sich nach dieser Zeit auch ein deutlicher Unterschied zwischen den mit P1 und P11 fermentierten Proben im Vergleich zur unfermentierten Mediumkontrolle ab. Es zeigte sich also, dass durch die Fermentation mit den Stämme P1 und P11 die Hexanal-Konzentration in dem Medium deutlich reduziert werden konnte.

### Probefermentationen

Es wurden verschiedene Probefermentationen durchgeführt (JC 18 *- Streptococcus thermophilus*, isoliert aus Erbsenmaterial, Ref - Fermentation mit kommerzieller Starterkultur, Mix 1 - Fermentation mit Stamm P1, Mix 2 - Fermentation mit einem anderen Stamm aus Erbsenmaterial, Mix 3 - Fermentation mit einem anderen Stamm aus Erbsenmaterial, Mix 4 - Fermentation mit einem anderen Stamm aus Erbsenmaterial, Mix 5 - Fermentation mit einem anderen Stamm aus Erbsenmaterial, Mix 6 - Fermentation mit Stamm P11) und jeweils der relative Hexanalgehalt bestimmt.

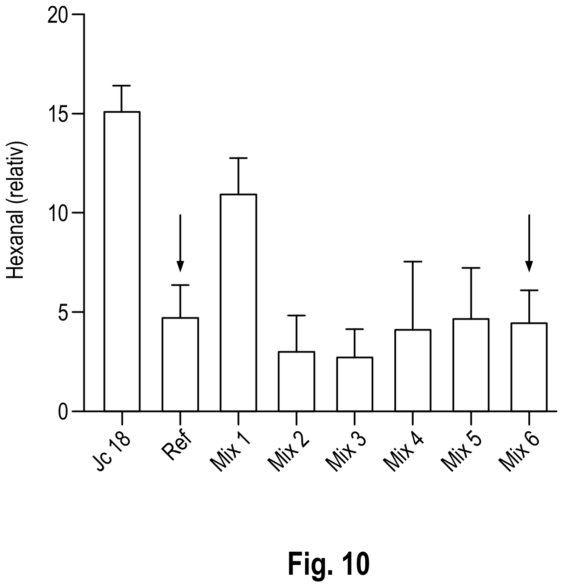

**Fig. 10** zeigt die Ergebnisse der Hexanalbestimmung. Es zeigte sich in der ersten Runde mit Probefermentationen, dass der Stamm P11 in Co-Kultur mit einem schwach ansäuernden *Streptococcus thermophilus*-Stamm (Mix 6) den Hexanalgehalt ähnlich gut absenken konnte wie die kommerzielle Referenz.

Die Kultur in Mix 1 beinhaltete P1 und den schwach ansäuernden *Streptococcus thermophilus-*Stamm. Wie in Fig. 10 zu sehen ist, war der Hexanalgehalt in dieser Probe höher als in der Referenzprobe.

Die fermentierten Proben wurden zusätzlich zu den chemischen Analysen verkostet (siehe Tabelle 3). Um den Einfluss der Starterkulturen besser bewerten zu können, wurden dem Grundrezept der "Ausgangserbsenmilch" keinerlei Maskierungs- oder Joghurtaromen beigesetzt, weshalb die Bewertung der Referenzprobe auch eher negativ ausfiel.

**Tabelle 3: Übersicht über Aroma- und Geschmackseindrücke, die bei der Verkostung der Proben entstanden. Pappig bezieht sich in diesem Kontext auf einen "pappe-artigen" Geschmack.**

| **Probe** | **Aromaeindrücke** | **Geschmackseindrücke** |
|---|---|---|
| Ref | Bohnig, grasig | Bitter, pappig, metallisch |
| **Mix 1** | Etwas bohnig | **Besser als Ref., milchig** |
| Mix 2 | Etwas bohnig | Relativ neutral, bitter, pappig |
| Mix 3 | Bohnig, grasig | Metallisch, astringierend, bitter |
| Mix 4 | bohnig | Umami, salzig |
| Mix 5 | bohnig, grasig | Zu salzig, pappig |
| **Mix 6** | Neutral, etwas sauer | **Milchig, angenehm säuerlich, etwas pappig** |

Insgesamt hatten Mix 1 (Stamm P1) und Mix 6 (Stamm P11) bei der Verkostung das beste sensorische Profil. Diese Ergebnisse zeigen, dass die Fermentation mit dem Stamm P1 und die Fermentation mit dem Stamm P11 das beste sensorische Ergebnis im Vergleich mit anderen Stämmen, die aus Erbsenmaterial isoliert worden waren, und im Vergleich mit einer herkömmlichen Starterkultur (Ref) erzielten. In Bezug auf den Hexanalgehalt war der Stamm P1 etwas schlechter als die herkömmliche Starterkultur. Der Stamm P11 erzielte jedoch eine mit der Referenz vergleichbare Verringerung des Hexanalgehalts. Durch eine Kombination der Stämme P1 und P11 zusammen mit einem ansäuernden *Streptococcus thermophilus-*Stamm lassen sich daher eine gute Hexanal-Reduktion bei gleichzeitig überlegenem sensorischem Profil erreichen.

## Patentansprüche

1. Starterkultur für die Fermentation einer pflanzlichen Milchalternative, insbesondere zur Herstellung eines pflanzlichen Joghurtalternativproduktes, **dadurch gekennzeichnet, dass** die Starterkultur Milchsäurebakterien umfasst, die aus Hülsenfrüchtler-Pflanzenmaterial, insbesondere aus Erbsenpflanzenmaterial, isoliert sind.

2. Starterkultur nach Anspruch 1, **dadurch gekennzeichnet, dass** die Milchsäurebakterien Vertreter der Art *Lactobacillus rhamnosus* und/oder der Art *Lactococcus lactis* ssp. *cremoris* sind.

3. Starterkultur nach Anspruch 1 oder Anspruch 2, **dadurch gekennzeichnet, dass** die Starterkultur Milchsäurebakterien der Art *Lactobacillus rhamnosus* umfasst, die Vertreter des Stammes P11 (Hinterlegungsstelle: DSMZ, Aktenzeichen der Hinterlegung: DSM 35210) sind.

4. Starterkultur nach einem der vorhergehenden Ansprüche, **dadurch gekennzeichnet, dass** die Starterkultur Milchsäurebakterien der Art *Lactococcus lactis* ssp. *cremoris* umfasst, die Vertreter des Stammes P1 (Hinterlegungsstelle: DSMZ, Aktenzeichen der Hinterlegung: DSM 35209) sind.

5. Starterkultur nach einem der vorhergehenden Ansprüche, **dadurch gekennzeichnet, dass** die Starterkultur zusätzlich Milchsäurebakterien der Art *Streptococcus thermophilus* umfasst.

6. Starterkultur nach einem der vorhergehenden Ansprüche, **dadurch gekennzeichnet, dass** die pflanzliche Milchalternative auf der Basis von Erbsenprotein und/oder Ackerbohnenprotein hergestellt ist.

7. Verfahren zur Herstellung eines fermentierten pflanzlichen Milchalternativproduktes, **dadurch gekennzeichnet, dass** das pflanzliche Milchalternativprodukt unter Verwendung einer Starterkultur gemäß einem der Ansprüche 1 bis 6 fermentiert ist.

8. Verfahren nach Anspruch 7, **dadurch gekennzeichnet, dass** das pflanzliche Milchalternativprodukt ein Joghurtalternativprodukt ist.

9. Verfahren nach Anspruch 7 oder Anspruch 8, umfassend die folgenden Verfahrensschritte:
a. Bereitstellen einer pflanzlichen Milchalternative,
b. Zusetzen der Starterkultur zu der pflanzlichen Milchalternative,
c. Fermentation der pflanzlichen Milchalternative,
d. Gegebenenfalls weitere Bearbeitung des pflanzlichen Milchalternativproduktes,
e. Abfüllung und Verpackung des pflanzlichen Milchalternativproduktes.

10. Fermentiertes pflanzliches Milchalternativprodukt, **dadurch gekennzeichnet, dass** das fermentierte pflanzliche Milchalternativprodukt unter Verwendung einer Starterkultur gemäß einem der Ansprüche 1 bis 6 fermentiert ist.

11. Fermentiertes pflanzliches Milchalternativprodukt nach Anspruch 10, **dadurch gekennzeichnet, dass** das fermentierte pflanzliche Milchalternativprodukt ein Joghurtalternativprodukt ist.

12. Fermentiertes pflanzliches Milchalternativprodukt nach Anspruch 10 oder Anspruch 11, **dadurch gekennzeichnet, dass** das fermentierte pflanzliche Milchalternativprodukt durch die Fermentation eine Verdickung und/oder eine Ansäuerung und/oder einen reduzierten Gehalt von *Off-Flavour-*Geschmacksstoffen und/oder einen erhöhten Gehalt von Milch- und/oder Joghurt-Aromastoffen und/oder einen reduzierten Gehalt von antinutritiven Inhaltsstoffen aufweist.

13. Verfahren zur Bereitstellung einer Starterkultur mit Mikroorganismen für die Fermentation einer pflanzlichen Milchalternative auf der Basis einer pflanzlichen Proteinquelle aus einem definierten pflanzlichen Material, **dadurch gekennzeichnet, dass** das definierte pflanzliche Material für die Isolierung der Mikroorganismen eingesetzt wird.

14. Verfahren nach Anspruch 13, umfassend die folgenden Verfahrensschritte:
a. Bereitstellen einer Probe des definierten pflanzlichen Materials,
b. Anreicherung und Isolierung von Mikroorganismen, insbesondere von grampositiven Bakterien, aus der Probe des pflanzlichen Materials,
c. Gegebenenfalls genotaxonomische Identifizierung der Mikroorganismen,
d. Gegebenenfalls phänotypische Charakterisierung der Mikroorganismen,
e. Durchführung von Probefermentationen der pflanzlichen Milchalternative mit den isolierten Mikroorganismen,
f. Auswahl und Zusammenstellung von geeigneten Mikroorganismen auf der Basis der Ergebnisse der Probefermationen für die Bereitstellung einer Starterkultur.

15. Verfahren nach Anspruch 14, **dadurch gekennzeichnet, dass** die phänotypische Charakterisierung der Mikroorganismen mindestens einen der folgenden Punkte umfasst:
a. Verdickung einer Matrix,
b. Ansäuerung einer Matrix,
c. Abbau von *Off-Flavour-*Geschmacksstoffen*,* insbesondere von Hexanal,
d. Produktion von Milch und/oder Joghurt-Aromastoffen, insbesondere von Acetaldehyd und/oder Diacetyl und/oder Acetoin,
e. Verwertbarkeit von verschiedenen Zuckerquellen,
f. Einfluss von verschiedenen Zuckerquellen auf eine Metabolitenproduktion.
